Europäisches Patentamt

European Patent Office

Office européen des brevets

(19)

(11) Veröffentlichungsnummer: **0 018 353**
**B1**

(12) **EUROPÄISCHE PATENTSCHRIFT**

(45) Veröffentlichungstag der Patentschrift:
**25.01.84**

(51) Int. Cl.³: **A 61 K 35/16**, C 12 Q 1/56,
G 01 N 33/86

(21) Anmeldenummer: **80890044.3**

(22) Anmeldetag: **15.04.80**

(54) **Verfahren zur Herstellung von nebenwirkungsfreien Plasmafraktionen.**

(30) Priorität: **19.04.79 AT 2940/79**

(43) Veröffentlichungstag der Anmeldung:
**29.10.80 Patentblatt 80/22**

(45) Bekanntmachung des Hinweises auf die Patenterteilung:
**25.01.84 Patentblatt 84/4**

(84) Benannte Vertragsstaaten:
**BE CH DE FR GB IT LI LU NL SE**

(56) Entgegenhaltungen:
**FR - A - 2 184 898**
**GB - A - 2 001 528**
**US - A - 4 137 223**

**CHEMICAL ABSTRACTS, Band 84, Nr. 7, 16. Februar 1976, Zusammenfassung Nr. 40350e, Seite 188, Columbus, Ohio, US, J.S. ROSENBERG et al.: "Inhibition of human factor IX, by human antithrombin"**
**CHEMICAL ABSTRACTS, Band 89, Nr. 7, 14. August 1978, Zusammenfassung Nr. 55381t, Seite 213, Columbus, Ohio, US, S. CHANDRA et al.: "Large scale preparation of nonthrombogenic prothrombin complex"**

**Die Akte enthält technische Angaben, die nach dem Eingang der Anmeldung eingereicht wurden und die nicht in dieser Patentschrift enthalten sind.**

(73) Patentinhaber: **IMMUNO Aktiengesellschaft für chemisch-medizinische Produkte, Industriestrasse 72, 1220 Wien (AT)**

(72) Erfinder: **Eibl, Johann, Dr., Gustav Tschermakgasse 2, A-1180 Wien (AT)**
Erfinder: **Elsinger, Fritz, Dr., Eduard Klein Gasse 29, A-1130 Wien (AT)**
Erfinder: **Linnau, Yendra, Dr., Lavendelweg 24, A-1220 Wien (AT)**

(74) Vertreter: **Wolfram, Gustav, Dipl.-Ing., Schwindgasse 7 P.O. Box 205, A-1041 Wien (AT)**

Verfahren zur Herstellung von nebenwirkungsfreien Plasmafraktionen

Die Erfindung betrifft ein Verfahren zur Herstellung von nebenwirkungsfreien Plasmafraktionen, insbesondere von Gerinnungsfaktor-Präparaten aus Plasma oder Plasmarohfraktionen, durch Reinigung und stufenweise Anreicherung von Plasmaproteinen, wie durch Filtrieren, Kryopräzipitieren, Fällen, Adsorbieren, Ultrafiltrieren, Zentrifugieren und Lyophilisieren, in Gegenwart von calciumbindenden Antikoagulantien, wie Citrationen.

Bei der Lagerung und Aufarbeitung von menschlichem und tierischem Plasma werden eine Reihe von Enzymen wirksam, die u.a. zu Veränderungen, wie Abbau oder Aktivierung, von hochmolekularen Plasmaproteinen führen. Diese Veränderungen können zur Bildung von Nebenprodukten führen, die wegen ihrer biologischen Aktivität unerwünschte Nebenwirkungen in vivo und in vitro zur Folge haben.

Bei der Herstellung von Konzentraten hochwirksamer Gerinnungsfaktoren und anderer Plasmaproteine mit biologischer Wirksamkeit ist die Gefahr enzymatisch bedingter Proteinveränderungen, die zu unerwünschten Nebenreaktionen führen können, besonders gross.

Es ist bereits bekannt, bei der Gewinnung des Prothrombin-Komplexes aus Plasma zum Zweck der Inaktivierung von Thrombin der Elutionslösung Antithrombin III zuzufügen (Sudhish Chandra und Milan Wickerhauser, Large Scale Preparation of Nonthrombogenic Prothrombin Complex, Thrombosis. Research, 12, 571 [1978]).

Weiter ist bekannt, bei der Herstellung des Prothrombin-Komplexes Heparin dem Endprodukt zuzugeben, um eine Prothrombin-Aktivierung zu verhindern (New England Journal of Medicine, 280, 291 [1969]).

Nach anderen Autoren (Jean-Pierre Soulier und Marion Steinbuch, Zeitschr. Ges. Inn. Med. 20, Suppl., 311, [1965]) hat Heparin eine stabilisierende Wirkung auf den Prothrombin-Komplex. Auch D. Ménaché und H. Roberts schlagen gemäss Throm. Diath. haemorrh. (Stuttgart) 33, 645 (1975), den Zusatz von Heparin zu Prothrombin-Komplexpräparaten vor.

Schliesslich ist gemäss der DE-OS 2 324 717 bei der Fraktionierung von Faktor VIII-Präparaten die Zugabe von Heparin bekannt, um die Ausbeute an Faktor VIII zu erhöhen.

Obwohl also bei der Herstellung von Gerinnungsfaktor-Präparaten sowohl die Zugabe des Antithrombin als auch die Zugabe von Heparin von mehreren Autoren vorgeschlagen worden ist, ist es bisher nicht gelungen, nebenwirkungsfreie Plasmafraktionen menschlichen oder tierischen Ursprungs, insbesondere nebenwirkungsfreie Gerinnungsfaktor-Präparate mit gleichmässiger Wirkung – in zuverlässiger und wiederholbarer Weise – herzustellen, und es besteht weiterhin das Bedürfnis, diese Aufgabe zu lösen.

Es wurde nun gefunden, dass für die Verhinderung der unerwünschten biologischen Veränderungen im Plasma bzw. in Plasmafraktionen nicht Antithrombin oder Heparin als solches massgeblich und wirksam ist, sondern dass es auf die Gegenwart einer Komplexverbindung aus Antithrombin III und Heparin bzw. Heparinoiden ankommt.

Das erfindungsgemässe Verfahren besteht darin, dass die Reinigung und Anreicherung der Plasmaproteine, wie der Gerinnungsfaktoren in Gegenwart einer Komplexverbindung aus Antithrombin III und Heparin bzw. Heparinoiden durchgeführt wird, wobei während aller Stufen des Fraktionierungsprozesses eine Konzentration an der Komplexverbindung von 0,5 bis 50 Einheiten ($AT_{av}$-E) pro ml der jeweiligen Lösung aufrecht erhalten wird.

Es wurde nämlich gefunden, dass es nicht ausreicht, ein stabilisierendes Mittel einfach dem Endprodukt oder mehr oder weniger willkürlich in irgendeiner Zwischenstufe des Fraktionierungsprozesses zuzufügen, wie dies dem aufgezeigten Stand der Technik eigen ist, sondern die Konzentration an der Komplexverbindung aufrecht erhalten werden.

Als eine Einheit der Komplexverbindung aus Antithrombin III und Heparin bzw. Heparinoiden, die auch als «avides Antithrombin», abgekürzt $AT_{av}$, bezeichnet wird, wird jene Menge definiert, welche eine NIH (National Institute of Health)-Einheit Thrombin in einer Minute inhibiert. Zur Bestimmung werden 2 NIH-Einheiten Thrombin mit der auf $AT_{av}$ zu testenden Probe bei 37 °C inkubiert. Nach einer Minute wird dem Inkubationsgemisch ein thrombinsensitives chromogenes Substrat auf Peptidbasis (z.B. Substrat S-2238 der Firma Kabi) zugefügt und die nicht inhibierte Menge Thrombin photometrisch bestimmt.

Das erfindungsgemässe Verfahren kann in verschiedener Weise ausgeübt werden. Eine vorteilhafte Ausführungsform der Erfindung besteht darin, dass die Komplexverbindung aus Antithrombin III und Heparin bzw. Heparinoiden in situ durch Zugabe von Heparin oder Heparinoiden gebildet wird.

Eine besonders bevorzugte und wichtige Ausführungsform des erfindungsgemässen Verfahrens besteht darin, dass die Komplexverbindung aus Antithrombin III und Heparin bzw. Heparinoiden vorgebildet wird und als solche während der einzelnen Stufen der Fraktionierung portionenweise zugefügt wird.

Das erfindungsgemässe Verfahren wird durch die folgenden Beispiele näher erläutert:

Beispiel 1:

Herstellung eines Faktor VII-Konzentrates 1 l frisch gefrorenes humanes Citratplasma wurde bei 0 bis 2 °C aufgetaut und das dabei anfallende Kryopräzipitat durch Zentrifugieren abgetrennt. In dem den Faktor VII enthaltenden Überstand (Kryoüberstand) wurden durch Zusatz von 0,5 Internationalen Einheiten (IE) Heparin pro ml 7 Einheiten der Komplexverbindung aus Antithrombin III und Heparin pro ml (7 $AT_{av}$-E/ml) in situ gebildet. Nach 15 Minuten wurden dem Kryoüberstand unter Rühren 0,5 g DEAE-Sephadex (diäthylaminoäthylgruppenhaltiges quervernetztes Dextran) zugesetzt: nach weiterem 30minütigem Rühren bei einer Temperatur von nicht über 5°C wird das DEAE-Sephadex durch Sedimentation oder Zentrifugieren abgetrennt. In dem DEAE-Sephadex-Überstand wurden wieder durch Zusatz von 0,5 IE Heparin/ml 7 $AT_{av}$-E/ml in situ gebildet. Nach 15 Minuten wurden dem DEAE-Sephadex-Überstand unter Rühren 7 ml einer 5%igen Suspension von Aluminiumhydroxid zugefügt; nach weiterem 15minütigem Rühren bei einer Temperatur unter 5°C wurde das Aluminiumhydroxid durch Zentrifugieren abgetrennt. Das den Faktor VII in adsorbierter Form enthaltende Aluminiumhydroxid wurde nun mit 100 ml (1/10 des ursprünglichen Plasmavolumens) einer Waschlösung behandelt, die 4 g/l Trinatriumcitrat · 2 $H_2O$ und 7 g/l Natriumchlorid enthielt. Weiters wurden der Lösung 10 Einheiten/ml vorgebildete Komplexverbindung aus Antithrombin III und Heparin (10 $AT_{av}$-E/ml) zugefügt. Die Vorbildung der Komplexverbindung erfolgte ausserhalb des eigentlichen Fraktionierungsprozesses durch Zusammenfügen einer Lösung von gereinigtem Antithrombin III und einer Heparinlösung, wobei in der resultierenden Lösung 0,5 Einheiten gereinigtes Antithrombin III und 0,5 IE Heparin pro ml enthalten waren. Nach 10minütigem Rühren bei 5°C wurde das gewaschene Aluminiumhydroxid durch Zentrifugieren abgetrennt und der Faktor VII bei erhöhter Ionenstärke eluiert. Zu diesem Zweck wurde das Aluminiumhydroxid mit 50 ml (1/20 des ursprünglichen Plasmavolumens) einer Elutionslösung behandelt, die 45 g/l sekundäres Natriumphosphat ($Na_2HPO_4$ · 12 $H_2O$) und 4 g/l Trinatriumcitrat · 2 $H_2O$ sowie 10 Einheiten/ml in dieser Lösung vorgebildete Komplexverbindung aus Antithrombin III und Heparin (10 $AT_{av}$-E/ml) enthielt. Die Vorbildung des Komplexes erfolgte wie vorstehend beschrieben. Der pH-Wert der Elutionslösung wurde mit einer sauren Phosphatlösung (78 g/l $NaH_2PO_4$ · $2H_2O$) auf 8,5 eingestellt. Nach 20minütigem Rühren bei 5°C wurde das Aluminiumhydroxid durch Zentrifugieren abgetrennt und verworfen. Das den Faktor VII enthaltende Eluat wurde mit der sauren Phosphatlösung auf einen pH-Wert von 7,5 eingestellt und hierauf bei 5°C gegen demineralisiertes Wasser dialysiert.

Das dialysierte Eluat wurde einer ersten Lyophilisation unterworfen; das so erhaltene Bulk wurde auf Faktor VII-Gehalt sowie mit Hilfe des im folgenden beschriebenen «NAPTT» (NON-ACTIVATED-PARTIAL-THROMBOPLASTIN-TIME)-Tests auf das Vorhandensein von aktivierten Gerinnungsfaktoren und auf den mit dem NAPTT-Test nicht erfassten, aktivierten Faktor VII (Faktor VIIa) getestet.

NAPTT-Test auf Anwesenheit von aktivierten Gerinnungsfaktoren:

a) Reagenzien

$a_1$) Normales humanes Citratplasma, nur mit Hilfe von Kunststoffgeräten gewonnen.

$a_2$) Partielles Thromboplastin (Phospholipid): «Tachostyptan» (Hormon Chemie, München), im Verhältnis 1:200 in «Tris» (Tris-Hydroxymethyl-Aminomethan)/NaCl Puffer verdünnt.

$a_3$) Puffer (als Verdünnungsmittel für Testproben und für Phospholipid):
0,06 m «Tris» - 7,26 g/l
0,09 m Natriumchlorid - 5,27 g/l
der pH-Wert wurde mit 1 n Salzsäure auf 7,5 eingestellt.

$a_4$) Kalziumchlorid: 0,025 m (m/40 $CaCl_2$).
Die $CaCl_2$-Lösung wurde während der Testperiode bei 37°C gehalten.

Die Testprobe und deren Verdünnungen sowie das Citratplasma ($a_1$) wurden während der Untersuchung im Eisbad aufbewahrt.

b) Testmethode:

Von der zu testenden Probe wurden unter Verwendung von «Tris»/NaCl Puffer 10 geometrische Verdünnungen (1:2, 1:4 bis 1:1024) in Kunststoffröhrchen hergestellt.

Diese Proben wurden zusammen mit einem «Leerwert» (als Probe wird der Puffer eingesetzt) in Doppelbestimmungen gemäss folgendem Verfahren getestet (der «Leerwert» wird am Anfang und am Ende der Testperiode bestimmt):
0,1 ml normales humanes Citratplasma
0,1 ml partielles Thromboplastin
0,1 ml Probe
Eine Minute bei 37°C inkubieren
0,1 ml $^m$/40 $CaCl_2$

Die Zeit von der Calciumchloridzugabe bis zur Gerinnungsbildung wurde gemessen (Kippen der Teströhrchen im 37°C-Wasserbad oder Anwendung eines automatischen Koagulometers).

Falls die unverdünnte Probe bzw. die ersten Verdünnungen verlängerte Gerinnungszeiten gegenüber dem «Leerwert» verursachen, muss der Probe jene Menge Protaminsulfat zugesetzt werden, welche die verlängerten Gerinnungszeiten auf den kürzestmöglichen Wert reduziert.

c) Beurteilung des Testergebnisses:

Die zu testende Probe ist frei von aktivierten Gerinnungsfaktoren («nicht aktiviert»), wenn die Gerinnungszeiten nach Zugabe der Probe bzw. deren Verdünnungen nicht kürzer sind als die kürzeste Gerinnungszeit der vor und nach der Testperiode bestimmten «Leerwerte» (Toleranzgrenze: 10–20 Sekunden, entsprechend der Fehlergrenze der Methode; Leerwert: mindestens 200 Sekunden).

Test auf Anwesenheit von aktiviertem Faktor VII (Faktor VIIa):

Prinzip des Tests:

Faktor VIIa kann durch Inkubation mit Antithrombin III (AT III) und Heparin in der Kälte inhibiert werden; nativer, d.h. nicht aktivierter Faktor VII ist unter diesen Bedingungen stabil. Die Abnahme der Faktor VII-Aktivität einer Probe nach Inkubation mit Faktor VII-Mangelplasma (AT III-Quelle) und Heparin im Vergleich zur Faktor VII-

Aktivität derselben Probe nach Inkubation mit einem Puffer (Kontrolle), ist ein Massstab für die in der Probe vorhandene Menge Faktor VIIa.

Testansatz (in Plastikröhrchen):
1,0 ml Testprobe (Verdünnung mit Puffer auf 10 Einheiten Faktor VII pro ml)
0,1 ml Heparin (Verdünnung mit Puffer auf 100 Internationale Einheiten Heparin pro ml)
0,9 ml Faktor VII-Mangelplasma (AT III-Quelle)

Kontrollansatz (in Plastikröhrchen):
1,0 ml Testprobe (10 Einheiten Faktor VII pro ml) 1,0 ml Puffer (0,7% Natriumchlorid: 0,7% Natriumcitrat)

Beide Ansätze werden über Nacht im Eisschrank stehen gelassen. Am nächsten Tag wird von beiden Ansätzen die Faktor VII-Aktivität mit Hilfe eines üblichen Gerinnungstests unter Anwendung von Faktor VII-Mangelplasma bestimmt.

Beurteilung des Testergebnisses:
Die Abnahme bzw. Inhibition der Faktor VII-Aktivität der Probe im Testansatz gegenüber dem Kontrollansatz ist proportional dem Faktor VIIa-Gehalt der Probe.

Die Berechnung erfolgt nach folgender Formel:

$$\% \text{ Inhibition} = \left(1 - \frac{\text{Faktor VII-Aktivität Testansatz}}{\text{Faktor VII-Aktivität Kontrollansatz}}\right) \times 100$$

0% Inhibition: die Probe enthält nur nicht aktivierten Faktor VII.
100% Inhibition: die Probe enthält nur aktivierten Faktor VII (Faktor VIIa).
Die am Endprodukt des Beispieles 1 vorgenommenen Tests zeigten Abwesenheit von aktivierten Gerinnungsfaktoren. Das Bulkmaterial wurde gelöst, mit Natriumcitrat und Natriumchlorid versetzt, sterilfiltriert, unter sterilen Bedingungen in Endbehälter abgefüllt und schliesslich lyophilisiert.

Beispiel 2 (Vergleichsversuch):
In diesem Versuch wurde ein Faktor VII-Konzentrat im wesentlichen nach der im Beispiel 1 angegebenen Methode hergestellt, jedoch mit der Abänderung, dass die In-situ-Bildung von der Komplexverbindung aus Antithrombin III und Heparin im Kryoüberstand sowie die In-situ-Bildung im DEAE-Sephadex-Überstand weggelassen wurde, wobei jedoch infolge des natürlichen Antithrombin III-Gehaltes des Ausgangsmaterials eine geringe Thrombin-Inhibitionswirkung entsprechend 0,4 $AT_{av}$-E/ml bestimmbar war.

Es wurde keine vorgebildete Komplexverbindung der Waschlösung und der Elutionslösung zugesetzt. Der NAPTT-Test und der VIIa-Test waren positiv, d.h. das Endprodukt enthielt aktivierte Gerinnungsfaktoren. Der Vergleich der Eigenschaften der Produkte des Beispieles 1 und des Beispieles 2 ist in der folgenden Tabelle I zahlenmässig zu ersehen.

Tabelle I

| | $AT_{av}$-E/ml in | | | | Aktivierung der Endprodukte | |
|---|---|---|---|---|---|---|
| | Kryo-überstand | DEAE-Seph.-Überstand | Wasch-lösung | Elutions-lösung | Akt. Gerinnungs-faktoren (NAPTT-Test) | Faktor VIIa % Inhibition |
| Beispiel 1 | 7 | 7 | 10 | 10 | nicht aktiviert | 0% |
| Beispiel 2 | 0,4 | 0,4 | 0 | 0 | aktiviert | 54% |

Beispiel 3:
Herstellung von Faktor VIII-Konzentrat mit aus Plasma und Heparin vorgebildeter Komplexverbindung Antithrombin III/Heparin
10,8 l frisch gefrorenes humanes Citratplasma wurden bei 0 bis 2°C aufgetaut. Das dabei anfallende Kryopräzipitat in einer Menge von 100 g wurde zentrifugiert und in 400 ml eines 0,2%igen Citratpuffers bei einem pH-Wert von 7,5 und einer Temperatur von 37°C gelöst, nachdem der Citratlösung die vorgebildete Komplexverbindung zugefügt wurde. Die Vorbildung erfolgte durch Zumischen einer Lösung von 73,5 ml Humanplasma und 147 IE Heparin, so dass die Kryopräzipitatlösung 6,0 $AT_{av}$-E/ml enthielt. Zu der Kryopräzipitatlösung wurden 19,6 ml einer 3%igen $Al(OH)_3$-Suspension gegeben, die Mischung 30 Minuten lang bei Raumtemperatur gerührt und das $Al(OH)_3$ durch Zentrifugieren abgetrennt.
Dem Überstand wurde weitere vorgebildete Komplexverbindung, hergestellt durch Vermischen von 37 ml Humanplasma und 74 IE Heparin, zugefügt, um einen Teil der an $Al(OH)_3$ adsorbierten Komplexverbindung zu ersetzen und deren Konzentration in der Lösung weiterhin auf 6,0 $AT_{av}$-E/ml zu halten. In der nächsten Stufe wurden 75 ml einer 53%igen Äthanollösung zugefügt, um eine Konzentration von 7% Äthanol zu erreichen und dadurch einen Teil der inerten (nicht Faktor VIII-aktiven) Proteine auszufällen. Diese Proteine wurden abgetrennt und verworfen. Dem Überstand wurden 39 ml Äthanol zugefügt, wodurch eine Konzentration von 10% Äthanol in der Lösung erreicht wurde. Die Temperatur wurde auf −2°C gesenkt, wodurch die Hauptmenge der Faktor VIII enthaltenden Fraktion ausfiel. Diese Fraktion wurde abgetrennt und in 150 ml einer 0,5%igen Natriumchloridlösung mit einem Gehalt von 0,2% Citrat und 1,0% Glycin gelöst. Auch in dieser Stufe wurde der Natriumchloridlösung vorgebildete Komplexverbindung (27 ml Humanplasma und 54 IE Heparin) zugefügt, um die Konzentration mit 6,0 $AT_{av}$-E/ml konstant zu halten. Die Lösung wurde über Membranfilter bis zu einem Porendurchmesser von 0,2 μm filtriert, das Faktor VIII-Konzentrat in die Endbehälter abgefüllt und lyophilisiert.

Das Faktor VIII-Konzentrat wurde, wie in den Beispielen 1 und 2 beschrieben, auf Anwesenheit von aktivierten Gerinnungsfaktoren geprüft. Das Ergebnis war negativ, d. h. es war frei von aktivierten Gerinnungsfaktoren.

Beispiel 4:
Herstellung von Faktor VIII-Konzentrat mit aus gereinigtem Antithrombin III und Heparin vorgebildeter Komplexverbindung

10,8 l frisch gefrorenes humanes Citratplasma wurden bei 0 bis 2°C aufgetaut. Das dabei anfallende Kryopräzipitat in einer Menge von 100 g wurde zentrifugiert und in 400 ml eines 0,2%igen Citratpuffers bei einem pH-Wert von 7,5 und einer Temperatur von 37°C gelöst, nachdem der Citratlösung vorgebildete Komplexverbindung zugefügt wurde. Die Vorbildung erfolgte durch Zumischen einer Lösung von 73,5 Einheiten gereinigtem Antithrombin III und 147 IE Heparin, so dass die Kryopräzipitatlösung 6,0 $AT_{av}$-E/ml enthielt. Zu der Kryopräzipitatlösung wurden 19,6 ml einer 3%igen Al(OH)$_3$-Suspension gegeben, die Mischung 30 Minuten lang bei Raumtemperatur gerührt und das Al(OH)$_3$ durch Zentrifugieren abgetrennt.

Dem Überstand wurde weitere vorgebildete Komplexverbindung, hergestellt durch Vermischen von 37 Einheiten gereinigtem Antithrombin III und 74 IE Heparin, zugefügt, um einen Teil der an Al(OH)$_3$ adsorbierten Komplexverbindung zu ersetzen und deren Konzentration in der Lösung weiterhin auf 6,0 $AT_{av}$-E/ml zu halten. In der nächsten Stufe wurden 75 ml einer 53%igen Äthanollösung zugefügt, um eine Konzentration von 7% Äthanol zu erreichen und dadurch einen Teil der inerten (nicht Faktor VIII-aktiven) Proteine auszufällen. Diese Proteine wurden abgetrennt und verworfen. Dem Überstand wurden 39 ml Äthanol zugefügt, wodurch eine Konzentration von 10% Äthanol in der Lösung erreicht wurde. Die Temperatur wurde auf −2°C gesenkt, wodurch die Hauptmenge der Faktor VIII enthaltenden Fraktion ausfiel. Diese Fraktion wurde abgetrennt und in 150 ml einer 0,5%igen Natriumchloridlösung mit einem Gehalt von 0,2% Citrat und 1,0% Glycin gelöst. Auch in dieser Stufe wurde der Natriumchloridlösung vorgebildete Komplexverbindung (27 Einheiten gereinigtes Antithrombin III und 54 IE Heparin) zugefügt, um die Konzentration mit 6,0 $AT_{av}$-E/ml konstant zu halten. Die Lösung wurde über Membranfilter bis zu einem Porendurchmesser von 0,2 μm filtriert, das Faktor VIII-Konzentrat in die Endbehälter abgefüllt und lyophilisiert.

Das Faktor VIII-Konzentrat wurde, wie in den Beispielen 1 und 2 beschrieben, auf Anwesenheit von aktivierten Gerinnungsfaktoren geprüft. Das Ergebnis war negativ, d. h. es war frei von aktivierten Gerinnungsfaktoren.

Beispiel 5:
Herstellung von Faktor VIII-Konzentrat mit aus Plasma und Heparinoid vorgebildeter Komplexverbindung Antithrombin III/Heparinoid

10,8 l frisch gefrorenes humanes Citratplasma wurden bei 0 bis 2°C aufgetaut. Das dabei anfallende Kryopräzipitat in einer Menge von 100 g wurde zentrifugiert und in 400 ml eines 0,2%igen Citratpuffers bei einem pH-Wert von 7,5 und einer Temperatur von 37°C gelöst, nachdem der Citratlösung vorgebildete Komplexverbindung zugefügt wurde. Die Vorbildung erfolgte durch Zumischen einer Lösung von 73,5 ml Humanplasma und 1470 Einheiten Eleparon$^R$ (Heparinoid: Mucopolysaccharidpolyschwefelsäureester), so dass die Kryopräzipitatlösung 1,5 $AT_{av}$-E/ml enthielt. Zu der Kryopräzipitatlösung wurden 19,6 ml einer 3%igen Al(OH)$_3$-Suspension gegeben, die Mischung 30 Minuten lang bei Raumtemperatur gerührt und das Al(OH)$_3$ durch Zentrifugieren abgetrennt.

Dem Überstand wurde weitere vorgebildete Komplexverbindung, hergestellt durch Vermischen von 37 ml Humanplasma und 740 Einheiten Eleparon$^R$, zugefügt, um einen Teil der an Al(OH)$_3$ adsorbierten Komplexverbindung zu ersetzen und deren Konzentration in der Lösung weiterhin auf 1,5 $AT_{av}$-E/ml zu halten. In der nächsten Stufe wurden 75 ml einer 53%igen Äthanollösung zugefügt, um eine Konzentration von 7% Äthanol zu erreichen und dadurch einen Teil der inerten (nicht Faktor VIII-aktiven) Proteine auszufällen. Diese Proteine wurden abgetrennt und verworfen. Zum Überstand wurden 39 ml Äthanol zugefügt, wodurch eine Konzentration von 10% Äthanol in der Lösung erreicht wurde. Die Temperatur wurde auf −2°C gesenkt, wodurch die Hauptmenge der Faktor VIII enthaltenden Fraktion ausfiel. Diese Fraktion wurde abgetrennt und in 150 ml einer 0,5%igen Natriumchloridlösung mit einem Gehalt von 0,2% Citrat und 1,0% Glycin gelöst. Auch in dieser Stufe wurde der Natriumchloridlösung vorgebildete Komplexverbindung (27 ml Humanplasma und 540 Einheiten Eleparon$^R$) zugefügt, um die Konzentration mit 1,5 $AT_{av}$-E/ml konstant zu halten. Die Lösung wurde über Membranfilter bis zu einem Porendurchmesser von 0,2 μm filtriert, das Faktor VIII-Konzentrat in die Endbehälter abgefüllt und lyophilisiert.

Das Faktor VIII-Konzentrat wurde, wie in den Beispielen 1 und 2 beschrieben, auf Anwesenheit von aktivierten Gerinnungsfaktoren geprüft. Das Ergebnis war negativ, d. h. es war frei von aktivierten Gerinnungsfaktoren.

Beispiel 6:

Herstellung von Faktor VIII-Konzentrat mit aus gereinigtem Antithrombin III und Heparinoid vorgebildeter Komplexverbindung

10,8 l frisch gefrorenes humanes Citratplasma wurden bei 0 bis 2 °C aufgetaut. Das dabei anfallende Kryopräzipitat in einer Menge von 100 g wurde zentrifugiert und in 400 ml eines 2%igen Citratpuffers bei einem pH-Wert von 7,5 und einer Temperatur von 37 °C gelöst, nachdem der Citratlösung vorgebildete Komolexverbindung zugefügt wurde. Die Vorbildung erfolgte durch Zumischen einer Lösung von 73,5 Einheiten gereinigtem Antithrombin III und 1470 Einheiten Eleparon$^R$, so dass die Kryopräzipitatlösung 1,5 AT$_{av}$-E/ml enthielt. Zu der Kryopräzipitatlösung wurden 19,6 ml einer 3%igen Al(OH)$_3$-Suspension gegeben, die Mischung 30 Minuten lang bei Raumtemperatur gerührt und das Al(OH)$_3$ durch Zentrifugieren abgetrennt.

Dem Überstand wurde weitere vorgebildete Komplexverbindung, hergestellt durch Vermischen von 37 Einheiten gereinigtem Antithrombin III und 740 Einheiten Eleparon$^R$, zugefügt, um einen Teil der an Al(OH)$_3$ adsorbierten Komplexverbindung zu ersetzen und deren Konzentration in der Lösung weiterhin auf 1,5 AT$_{av}$-E/ml zu halten. In der nächsten Stufe wurden 75 ml einer 53%igen Äthanollösung zugefügt, um eine Konzentration von 7% Äthanol zu erreichen und dadurch einen Teil der inerten (nicht Fraktor VIII-aktiven) Proteine auszufällen. Diese Proteine wurden abgetrennt und verworfen. Zum Überstand wurden 39 ml Äthanol zugefügt, wodurch eine Konzentration von 10% Äthanol in der Lösung erreicht wurde. Die Temperatur wurde auf −2 °C gesenkt, wodurch die Hauptmenge der Faktor VIII enthaltenden Fraktion ausfiel. Diese Fraktion wurde abgetrennt und in 150 ml einer 0,5%igen Natriumchloridlösung mit einem Gehalt von 0,2% Citrat und 1,0% Glycin gelöst. Auch in dieser Stufe wurde der Natriumchloridlösung vorgebildete Komplexverbindung (27 Einheiten gereinigtes Antithrombin III und 540 Einheiten Eleparon$^R$) zugefügt, um die Konzentration mit 1,5 AT$_{av}$-E/ml konstant zu halten. Die Lösung wurde über Membranfilter bis zu einem Porendurchmesser von 0,2 µm filtriert, das Faktor VIII-Konzentrat in die Endbehälter abgefüllt und lyophilisiert.

Das Faktor VIII-Konzentrat wurde, wie in den Beispielen 1 und 2 beschrieben, auf Anwesenheit von aktivierten Gerinnungsfaktoren geprüft. Das Ergebnis war negativ, d. h. es war frei von aktivierten Gerinnungsfaktoren.

Beispiel 7:

Herstellung von Faktor VIII-Konzentrat mit Heparinzusatz, jedoch ohne Antithrombin III-Zusatz, zu einer Plasmafraktion (Vergleichsversuch)

10,8 l frisch gefrorenes humanes Citratplasma wurden bei 0 bis 2 °C aufgetaut. Das dabei anfallende Kryopräzipitat in einer Menge von 100 g wurde zentrifugiert und in 400 ml eines 0,2%igen Citratpuffers bei einem pH-Wert von 7,5 und einer Temperatur von 37 °C gelöst. Zu der Kryopräzipitatlösung wurden 19,6 ml einer 3%igen Al(OH)$_3$-Suspension gegeben, die Mischung 30 Minuten lang bei Raumtemperatur gerührt und das Al(OH)$_3$ durch Zentrifugieren abgetrennt.

Dem Überstand wurden 75 ml einer 53%igen Äthanollösung zugefügt, um eine Konzentration von 7% Äthanol zu erreichen und dadurch einen Teil der inerten (nicht Faktor VIII-aktiven) Proteine auszufällen. Diese Proteine wurden abgetrennt und verworfen. Dem Überstand wurden 39 ml Äthanol zugefügt, wodurch eine Konzentration von 10% Äthanol in der Lösung erreicht wurde. Die Temperatur wurde auf −2 °C gesenkt, wodurch die Hauptmenge der Faktor VIII enthaltenden Fraktion ausfiel. Diese Fraktion wurde abgetrennt und in 150 ml einer 0,5%igen Natriumchloridlösung mit einem Gehalt von 0,2% Citrat und 1,0% Glycin gelöst. Der Natriumchloridlösung wurden 54 IE Heparin zugefügt. Die Lösung wurde über Membranfilter bis zu einem Porendurchmesser von 0,2 µm filtriert, das Faktor VIII-Konzentrat in die Endbehälter abgefüllt und lyophilisiert. Das Präparat wurde, wie vorher beschrieben, auf Anwesenheit von aktivierten Blutgerinnungsfaktoren geprüft. Dabei zeigte sich, dass die Zugabe von Heparin zur letzten Fraktion des Fraktionierungsprozesses nicht ausreichend war, um Aktivierungen zu verhindern. Der Aktivierungstest war positiv, d. h. es waren aktivierte Gerinnungsfaktoren vorhanden, und das Präparat musste verworfen werden.

Beispiel 8:

Herstellung von Faktor VIII-Konzentrat ohne Zusatz von Heparin und Antithrombin III (Vergleichsversuch)

10,8 l frisch gefrorenes humanes Citratplasma wurden bei 0 bis 2 °C aufgetaut. Das dabei anfallende Kryopräzipitat in einer Menge von 100 g wurde zentrifugiert und in 400 ml eines 0,2%igen Citratpuffers bei einem pH-Wert von 7,5 und einer Temperatur von 37 °C gelöst. Zu der Kryopräzipitatlösung wurden 19,6 ml einer 3%igen Al(OH)$_3$-Suspension gegeben, die Mischung 30 Minuten lang bei Raumtemperatur gerührt und das Al(OH)$_3$ durch Zentrifugieren abgetrennt.

Dem Überstand wurden 75 ml einer 53%igen Äthanollösung zugefügt, um eine Konzentration von 7% Äthanol zu erreichen und dadurch einen Teil der inerten (nicht Faktor VIII-aktiven) Proteine auszufällen. Diese Proteine wurden abgetrennt und verworfen. Dem Überstand wurden 39 ml Äthanol zugefügt, wodurch eine Konzentration von 10% Äthanol in der Lösung erreicht wurde. Die Temperatur wurde auf −2 °C gesenkt, wodurch die Hauptmenge der Faktor VIII enthaltenden Fraktion ausfiel. Diese Fraktion wurde abgetrennt und in 150 ml einer 0,5%igen Natriumchloridlösung mit einem Gehalt von 0,2% Citrat und 1,0% Glycin gelöst. Die Lösung wurde über Membranfilter bis zu einem Porendurchmesser von 0,2 µm filtriert, das Faktor VIII-Konzentrat in die Endbehälter abgefüllt und lyophilisiert.

Das Präparat wurde, wie vorher beschrieben, auf Anwesenheit von aktivierten Blutgerinnungsfaktoren geprüft. Der Aktivierungstest war positiv, d.h. es waren solche aktivierten Faktoren vorhanden und das Präparat musste verworfen werden.

Während im vorstehenden im einzelnen das erfindungsgemässe Verfahren für die Herstellung von Faktor VII- und Faktor VIII-Konzentraten erläutert wurde, ist es in gleicher Weise auch für die Herstellung von Faktor IX- bzw. Prothrombinkomplex-Konzentraten geeignet und vorteilhaft.

## Patentansprüche

1. Verfahren zur Herstellung von nebenwirkungsfreien Plasmafraktionen, insbesondere von Gerinnungsfaktor-Präparaten aus Plasma oder Plasmarohfraktionen, durch Reinigung und stufenweise Anreicherung von Plasmaproteinen, wie durch Filtrieren, Kryopräzipitieren, Fällen, Adsorbieren, Ultrafiltrieren, Zentrifugieren und Lyophilisieren, in Gegenwart von calciumbindenden Antikoagulantien, wie Citrationen, dadurch gekennzeichnet, dass die Reinigung und Anreicherung der Plasmaproteine, wie Gerinnungsfaktoren in Gegenwart einer Komplexverbindung aus Antithrombin III und Heparin bzw. Heparinoiden durchgeführt wird, wobei während aller Stufen des Fraktionierungsprozesses eine Konzentration an der Komplexverbindung von 0,5 bis 50 Einheiten ($AT_{av}$-E) pro ml der jeweiligen Lösung aufrechterhalten wird.

2. Verfahren nach Anspruch 1, dadurch gekennzeichnet, dass die Komplexverbindung in situ durch Zugabe von Heparin gebildet wird.

3. Verfahren nach Anspruch 1, dadurch gekennzeichnet, dass die Komplexverbindung vorgebildet wird und als solche während der einzelnen Stufen der Fraktionierung portionenweise zugefügt wird.

## Claims

1. A method of producing plasma fractions free of side-effects, in particular of coagulation-factor preparations from plasma or plasma crude fractions, by purification and stepwise enrichment of plasma proteins, such as by filtration, cryoprecipitation, precipitation, adsorption, ultrafiltration, centrifugation and lyophilisation, in the presence of calcium-binding anticoagulants, such as citrate ions, characterised in that the purification and enrichment of the plasma proteins, such as the coagulation factors, is carried out in the presence of a complex compound of antithrombin III and heparin or heparinoids, a concentration of the complex compound of 0.5 to 50 units ($AT_{av}$-U) per ml of the respective solution being maintained during all the steps of the fractionation process.

2. A method according to claim 1, characterised in that the complex compound is formed in situ by the addition of heparin.

3. A method according to claim 1, characterised in that the complex compound is preformed and as such is added in portions during the individual steps of the fractionation.

## Revendications

1. Procédé de préparation de fractions de plasma exemptes d'activités secondaires, en particulier de concentrés de facteurs de coagulation, à partir du plasma ou de fractions brutes de plasma, par purification et enrichissement progressif des protéines du plasma, par exemple par filtration, cryoprécipitation, précipitation, adsorption, ultrafiltration, centrifugation et lyophilisation, en présence d'anticoagulants fixant le calcium tels que les ions citrates, caractérisé en ce que la purification et l'enrichissement des protéines du plasma telles que les facteurs coagulants sont effectués en présence d'un complexe d'antithrombine III et d'héparine ou d'héparinoïdes, avec maintien dans tous les stades des opérations de fractionnement d'une concentration de 0,5 à 50 unités (U-$AT_{av}$) du complexe par ml de la solution particulière.

2. Procédé selon la revendication 1, caractérisé en ce que le complexe est formé in situ par addition d'héparine.

3. Procédé selon la revendication 1, caractérisé en ce que le complexe est formé au préalable et ajouté tel quel, par portions, dans les divers stades du fractionnement.